# EUROPEAN PATENT SPECIFICATION

(11) **EP 3 907 835 B1**
(45) Date of publication and mention of the grant of the patent: **03.08.2022**
(21) Application number: 20172968.8
(22) Date of filing: 05.05.2020
(51) Int. Cl.: H01S 3/0941, H01S 3/102, H01S 5/06, H01S 3/10, H01S 3/16, H01S 5/042, H01S 5/062, A61B 18/26, A61B 18/00

(54) **METHOD FOR OPERATING DIODE-PUMPED PULSED LASERS**
VERFAHREN ZUM BETREIBEN VON DIODENGEPUMPTEN GEPULSTEN LASERN
PROCÉDÉ DE FONCTIONNEMENT DE LASERS PULSÉS POMPÉS PAR DES DIODES

(43) Date of publication of application: 10.11.2021
(73) Proprietor: Dornier MedTech Laser GmbH, 82234 Wessling (DE)
(72) Inventor: Hiereth, Werner, 82234 Wessling (DE)
(74) Representative: Grünecker Patent- und Rechtsanwälte PartG mbB

(56) References cited:
- EP-A1- 2 443 707
- EP-A2- 1 696 522
- WO-A1-2013/079943
- US-A1- 2006 018 350
- US-A1- 2011 134 947
- US-B1- 9 368 933

## Description

### State of the art

The invention relates to a method as defined in patent claim 1 and a laser system as defined in patent claim 5.

Solid-state lasers are commonly pumped using gas lamps or laser diodes as pump light sources. While gas lamps are cheaper than laser diodes, they have a shorter lifetime and further can only provide a broad spectral emission, resulting in a rather low efficiency of the pumping of solid state lasers.

Diode lasers on the other hand are much more expensive than gas lamps but have a much higher lifetime and a small spectral width (e.g. 2-3 nm) of their emission spectrum and are therefore much more efficient in the pumping of solid state lasers having spectral absorption bands with narrow widths (e.g. a few nm). However, in order to efficiently pump solid state lasers with narrow width absorption bands, diode lasers must be operated in a rather small temperature range because the emitted wavelength / emitted spectrum of the diode laser is dependent on the temperature of the laser diode and, for example, the emitted wavelength or emission spectrum peak wavelength can change or shift, for example, by ~ 0,28 nm/°C.

Hence, laser diodes are designed for emitting a defined wavelength / defined emission spectrum peak wavelength only at a defined operating temperature.

While for continuous wave diode-pumped solid-state lasers (CWDPSSL) this is not a problem, since the laser diodes have a constant heat dissipation during operation, for pulsed diode-pumped solid-state lasers (PDPSSL), the temperature-wavelength relationship between the emitted wavelength of the laser diode and its temperature can inter alia depend on the duty cycle of operation and cooling parameters/operation of the laser diode.

Current techniques for pumping pulsed solid-state lasers are cumbersome, slow and not efficient, resulting, for example, in undesired reductions of the laser power output efficiency of the solid-state lasers over a broad range of desired operating frequencies of the solid-state laser. Furthermore, current techniques for pumping pulsed solid-state lasers via laser diodes suffer from the drawback that the range of temperatures in which the laser diode can operate is rather limited, thereby also further limiting the possible range of operating frequencies / frequency settings of pulsed solid-state laser, especially for medical / surgical applications.

In addition current solutions to optimize the control of the temperature of laser diodes, i.e. for controlling the emission spectrum / emitted wavelength of the laser diodes, for the pumping of solid-state lasers are rather static, have a low latency for compensation of laser diode temperature changes and inter alia require complex and dedicated cooling mechanisms for the laser diodes, e.g. comprising thermo-electric coolers.

WO 2013/079943 A1 or US 2006/018350 A1 discloses a pulsed diode-pumped solid-state laser wherein the laser diode pump light source is configured for emitting a series of light pulses for pumping the solid-state laser.

In WO 2013/079943 A1 a constant thermal lens inside the laser medium is kept stable by modulated optical pumping, wherein between the laser pulses, additional pulsed thermal compensation pump pulses are provided below threshold operation of the laser to keep the thermal lens inside the laser medium constant.

In US 2006/018350 A1 a burst pulse of pumping power is applied to the laser gain medium which heats the gain medium, and the gain medium is thereafter underheated sufficiently by applying an intermediate pulse to realize steady-state thermal load on the gain medium before applying a subsequent pulse. The solid-state gain medium may be Tm:Ho:YAG.

EP 2 443 707 A1 discloses a diode pumped solid-state laser, which according to one example is a Tm:YAG laser side pumped by a laser diode of pump wavelength 760nm-815nm. The solid-state laser may be operated from about 100Hz to 1000 Hz. In one example the the solid-state laser is operated at 250Hz with pulse lengths of 200 microseconds, and with driving current amplitude in the range of 50A to 220A.

### Problem

It is therefore the object of the present invention to provide improved means for pumping pulsed solid-state lasers. For example, it is in particular an object of the present invention to improve the efficiency and effectivity of diode-pumping a pulsed solid-state laser and to improve the stability and laser power output efficiency of a/the pulsed diode-pumped solid-state laser.

### Solution

According to the present invention, this object is achieved by a method according to claim 1 and a laser system according to claim 5.

Advantageous embodiments and further developments are the subject matter of the dependent claims.

An exemplary method for operating a pulsed diode-pumped solid-state laser in accordance with the present invention comprises all of the following steps:
- providing a pump light source for pumping a solid-state laser, wherein said pump light source comprises at least one laser diode unit configured for emitting a series of light pulses for pumping the solid-state laser,
- modulating the series of light emission pulses of the at least one laser diode unit such that only the light pulses with a frequency close to or equal to a requested frequency setting of the solid-state laser are operated with a/the required pulse amplitude and/or a/the required pulse duration to trigger light emission of the solid-state laser,
   and the at least one laser diode unit further being configured such that any other light pulses of the at least one laser diode unit are operated to not trigger light emission of the solid-state laser.

Herein, the terms required pulse amplitude and required pulse duration for triggering light emission of the solid-state laser can be inter alia understood as a required minimal pulse amplitude and as a required minimal pulse duration for triggering light emission of the solid-state laser.

Hence, in the modulation step exemplary described above, the light pulses with a frequency close to or equal to a requested frequency setting of the solid-state laser can be operated with pulse amplitudes that are equal to or larger than a/the required (minimal) pulse amplitude and/or with pulse durations that are equal to or larger than a/the required (minimal) pulse duration for triggering light emission of the solid-state laser.

Stated differently, the terms "required pulse amplitude" and "required pulse duration" can be understood as referring to a minimal pulse amplitude and/or a minimal pulse duration required to trigger light emission of the solid-state laser.

Furthermore, herein a frequency close to a requested frequency setting of the solid-state laser can inter alia be understood as a frequency within a predetermined interval around the requested frequency setting. For example, within an interval of +/- 10% or within an interval of +/- 20% of the requested frequency setting for the pulsed operation of the solid-state laser.

In other words, in the herein exemplary described method for operating a pulsed diode-pumped solid-state laser, only the by the at least one laser diode unit emitted light pulses with a frequency close to or equal to a requested frequency setting of the solid-state laser are operated with laser diode pumping parameters, such as, for example, pulse duration(s) and pulse amplitude(s), that are sufficient to trigger laser emission from the solid-state laser, i.e. only the by the at least one laser diode unit emitted light pulses with a frequency close to or equal to a requested frequency of the solid-state laser are provided with energies above the lasing threshold of the solid-state laser.

Any other light pulses emitted by the at least one laser diode unit, i.e. light pulses with frequencies not equal to a/the requested frequency of the solid-state laser and/or light pulses with frequencies outside, i.e. above or below, a/said predetermined interval around a/the requested frequency setting of the solid-state laser are operated with laser diode pumping parameters, such as, for example, pulse duration(s) and pulse amplitude(s), that do not trigger a light emission of the solid-state laser.

Stated differently, light pulses emitted by the at least one laser diode unit with frequencies not equal to a/the requested frequency of the solid-state laser and/or light pulses with frequencies outside, i.e. above or below, a/said predetermined interval around a/the requested frequency setting of the solid-state laser are operated / provided with energies below the lasing threshold of the solid-state laser.

Hence, between light pulses emitted by the solid-state laser, the at least one laser diode unit can continue injecting / pumping of the solid-state laser with light pulses, but at energies below the lasing threshold of the solid-state laser.

The herein exemplary described method steps for operating a pulsed diode-pumped solid-state laser and the herein exemplary described laser system and laser system configurations for operating a pulsed diode-pumped solid-state laser can inter alia significantly improve the laser power output of a solid-state laser operated in pulsed mode.

In-house experimental tests have surprisingly shown that significant increases of laser power output of a pulsed solid-state laser can be achieved. In particular, for example, increases in the maximum energy per laser pulse of up to 30% or more can be achieved as compared to known pulsed solid-state laser systems. This benefit is in particular notable in low frequency settings. The reasons for this rather unexpected and surprisingly large beneficial technical effect can inter alia be understood from the fact that, due to the modulated pulsed operation of the laser diode unit comprising pulses with energies below, the lasing threshold of the solid-state laser can heat up the laser diode(s) of the laser diode unit more quickly, more effectively and more efficiently to reach a/the designated operation temperature of the laser diode(s) of the laser diode unit, thus enabling a faster shift of the wavelength of the light emitted by the laser diode(s) of the laser diode unit towards / to the designated operating wavelength of the laser diode(s) of the laser diode unit at which the gain medium / the laser crystal of the solid-state laser best absorbs the light emitted by the laser diode(s) of the laser diode unit.

Not only is the designated operating temperature of the laser diode(s) of the laser diode unit reached faster as compared to current systems, but the operating conditions of the laser diode(s), in particular its/their operating temperature, is kept more stable and accurate as compared to current systems. For example, since the laser diode(s) of the laser diode unit are not operated at full capacity, maximum energy or maximum current at all times during the pumping operation, also the risk of overheating the laser diode(s) of the laser diode unit and thereby again the risk of shifting the wavelength of the pump light emitted by the laser diode(s) of the laser diode unit to wavelengths which are absorbed less or not absorbed at all by the gain medium / the laser crystal of the solid-state laser and resulting in inefficient pumping and loss in laser power output of the solid-state laser is reduced.

Consequently, the pumping of the solid state laser is more stable, more efficient and more accurate, resulting also in a more stable laser output of the solid-state laser.

Thanks to the rapid reach of optimal operations of the laser diode(s) of the laser diode unit to rapidly establish optimal and efficient pumping of the solid-state laser, the methods and means described herein also enable fast changes of different operation modes of the solid-state laser, e.g. fast changes between different frequency settings of the solid-state laser, such as, for example, changing from high frequency settings / high repetition rate settings to low frequency settings / low repetition rate settings. Herein, high frequency settings / high repetition rate settings may, for example, refer to frequencies greater than 100 Hz or greater than 200 Hz, and low frequency settings / low repetition rate settings may refer to frequencies less than 100 Hz or less than 50 Hz.

The herein exemplary presented method steps for operating a pulsed diode-pumped solid-state laser and the herein exemplary described laser system configurations allow quasi-instantaneous changes between different frequency settings of the solid-state laser, for example, between low frequency settings and high frequency settings. In other words, the herein exemplary presented method steps for operating a pulsed diode-pumped solid-state laser and the herein exemplary described laser system configurations essentially eliminate the rather long time scales needed in conventional systems, which may require tens of seconds or minutes or even longer, for changes between different frequency settings of the solid-state laser.

Since the herein exemplary presented method steps for operating a pulsed diode-pumped solid-state laser and the herein exemplary described laser system configurations allow for more efficient and stable operations of the pulsed diode-pumped solid-state laser system, for example, a cooling mechanism for the laser diodes can be simplified and, for example, can be implemented by a water cooling mechanism. Consequently, more expensive and/or more cumbersome thermal regulation components for the laser diode(s) of the laser diode unit, such as thermo-electric coolers, can be dispensed with.

Hence, aside from providing a faster operation flow of the solid-state laser, improved power output of the solid-state laser, in particular in low frequency settings higher laser energies per laser pulse can be achieved as compared to known solid-state laser systems, and lower latencies for changing between different frequency settings of the solid-state laser, the herein exemplary described laser system configurations can further allow a simplified and more compact design of a diode-pumped solid-state laser system as compared to current systems.

The herein exemplary described means, laser system configurations and methods for operating a diode-pumped solid-state laser can be in particular suited for pulsed diode-pumped solid-state lasers for medical/surgical applications, e.g. for the treatment of biological tissue, and wherein the solid-state laser is being operated in pulsed mode in a frequency settings range of one to several hundreds of Hz.

The selectable diode-pumped solid-state laser frequency setting in accordance with the invention lies at a frequency below 300 Hz, in particular, for example, at or below 100 Hz.

For example, for the treatment of soft tissue with the herein described solid-state laser system a frequency setting with a frequency between 100 - 300 Hz or between 50 - 300Hz may be applicable.

For the treatment or removal of harder components, such as urinary stones, for example, a frequency setting for the diode-pumped solid-state laser within the range of frequency settings from 3 - 100 Hz could be used.

Lower frequency settings for the diode-pumped solid-state laser together with higher energies / higher laser diode currents, e.g. with laser diode currents of up to 100 A or up to 200 A or higher, can be applied in order facilitate and speed up the fragmentations of stones that are to be removed from a patient. Such a treatment or operation mode can inter alia be referred to as fragmentation. Smaller stone fragments can then subsequently be removed using higher frequency settings for the diode-pumped solid-state laser and lower energies / lower laser diode currents, e.g. laser diode currents of less than 100 A or less than 50 A. Such a treatment or operation mode can inter alia be referred to as dusting.

An exemplary method for operating a pulsed diode-pumped solid-state laser then may comprise the following setups and steps.

For example, the selectable / requestable / target laser frequency setting for an exemplary diode-pumped solid-state laser can be one of the following frequency settings: 5, 10, 20, 25, 50, 75 or 100 Hz and an exemplary laser diode unit can be designed / configured for emitting light pulses with a frequency of 100 Hz for a chosen pumping wavelength, e.g. a pumping wavelength optimized for a preferred / desired frequency setting of the solid-state laser, i.e. a desired operating mode of the solid-state laser.

For said exemplary frequency settings of the diode-pumped solid-state laser, the modulation of the light pulses emitted from/by the exemplary laser diode unit can be specified as follows.
- For example, for a selected /requested solid-state laser frequency setting of 5 Hz, only every 20th light pulse of the laser diode unit can be operated with the required pulse amplitude and/or the required pulse duration to trigger light emission of the solid-state laser,
- for a selected /requested solid-state laser frequency setting of 10 Hz only every 10th light pulse of the laser diode unit can be operated with the required pulse amplitude and/or the required pulse duration to trigger light emission of the solid-state laser,
- for a selected /requested solid-state laser frequency setting of 20 Hz only every 5th light pulse of the laser diode unit can be operated with the required pulse amplitude and/or the required pulse duration to trigger light emission of the solid-state laser,
- for a selected /requested solid-state laser frequency setting of 25 Hz only every 4th light pulse of the laser diode unit is operated with the required pulse amplitude and/or the required pulse duration to trigger light emission of the solid-state laser,
- and wherein, in an example not in accordance with the invention, for a selected /requested solid-state laser frequency setting of 50 Hz only every 2nd light pulse of the laser diode unit is operated with the required pulse amplitude and/or the required pulse duration to trigger light emission of the solid-state laser,
- and wherein, in an example not in accordance with the invention, for a selected /requested solid-state laser frequency setting of 75 Hz or 100 Hz all pulses of the laser diode unit are operated with the required pulse amplitude and/or the required pulse duration to trigger light emission of the solid-state laser.

Herein, an exemplary required pulse duration to trigger light emission of the solid-state laser can be a duration of up to 150 µs or can be a duration of up to 500 µs or more and a required exemplary pulse amplitude expressed in terms of electrical current applied to drive a laser diode / the laser diode unit can be up to 200 A or more and an exemplary electrical current applied to a laser diode / the laser diode unit that results in an emission of light pulse from the laser diode / the laser diode unit and that does not trigger a laser light emission from the solid-state laser can lie for example at 65 A or below.

It is inter alia further conceivable that the required pulse amplitude and/or the required pulse duration to trigger light emission of the solid-state laser are further modulated such that the values of the required pulse amplitude(s) and/or the required pulse duration(s) of the pulses for triggering light emission of the solid-state laser can differ for a selected / requested solid-state laser frequency setting.

In examples not in accordance with the invention, in the exemplary cases listed above, it is conceivable that for a selected / requested solid-state laser frequency setting of 75 Hz or 100 Hz all pulses of the laser diode unit are operated with at least the required pulse amplitude and/or with at least the required pulse duration to trigger light emission of the solid-state laser, but that the pulses are not all having the same pulse amplitude and/or the same pulse duration. In other words in this example, the pulses may have at least an amplitude and a pulse duration required for triggering light emission of the solid-state laser but some or each may have different pulse amplitudes and/or different pulse durations.

It is further conceivable, that in general during operation of the pulsed diode-pumped solid-state laser, the electrical current provided to drive the at least one laser diode / laser diode unit can be non-zero at all times. This can inter alia further facilitate control of the operating temperature of the laser diode unit and, in particular, can further shorten the warm-up time of the laser diode unit and can therefore further optimize (i.e. reduce) latency of the laser system when operating the solid-state laser with / changing between different frequency settings.

An exemplary diode-pumped solid-state laser system that can be operated as exemplary described herein may comprise a solid-state laser and at least one laser diode unit that can be configured to operate according to any of the herein described exemplary described steps.

In accordance with the invention, the solid-state laser is a Tm:YAG (Thulium:Ytterium Aluminium Garnate) solid-state laser.

An exemplary laser diode unit, an exemplary laser diode of the laser diode unit, may be configured for emitting light pulses with a wavelength of 780 nm or in the range of 778 - 782 nm at 100 Hz and with pulse durations of up to 500 µs and with amplitudes of up to a maximum current of 200 A or 250 A and at an exemplary temperature of 25°C.

An exemplary laser diode unit may comprise a single laser diode or a one-dimensional array of laser diodes, e.g. a diode-laser bar, or a two-dimensional array of laser diodes, e.g. a stack of diode-laser bars, or a three-dimensional array of laser diodes, e.g. comprising multiple stacks of diode-laser bars.

The herein described exemplary laser diode unit / the exemplary laser diode(s) of the exemplary laser diode unit can in particular be configured such that it can emit light with an emission spectrum substantially matching the absorption spectrum of the gain medium of the solid-state laser, when the laser diode unit/the laser diode(s) is/are operated at or close to the maximum of a predetermined electrical current supplied to the laser diode unit/the laser diode(s).

In particular, the herein described exemplary laser diode unit / the exemplary laser diode(s) of the exemplary laser diode unit can be configured / designed such as to emit light at / with a wavelength matching the wavelength at which the gain medium of the solid-state laser best absorbs light when the laser diode unit / the exemplary laser diode(s) is/are operated at/with a predetermined maximum electrical current and with/at a frequency (a/the operating frequency) which is the most critical for the intended use, i.e. the requested frequency setting *f* of the solid-state laser in terms of maximum laser power output / laser energy output E, such that the product *E* × *f* can be optimized.

Furthermore, the solid-state laser can be operated at/with a requested frequency setting as close as possible (e.g. within less than 10 or 20%) to a/the designed operating frequency of an exemplary laser diode unit / exemplary laser diode(s) to optimize the laser power output / laser energy output of the solid-state laser. Herein, the operating frequency of the exemplary laser diode unit / the exemplary laser diode(s) can be inter alia understood as pumping frequency of providing / emitting light pulses from the exemplary laser diode unit / the exemplary laser diode(s) to the solid-state laser that do not trigger a laser emission of the solid-state laser.

The following figures illustrate exemplary:
**Fig. 1****:** Exemplary modulation of a series of light emission pulses of a laser diode unit
**Fig. 2****:** Exemplary solid-state laser power/energy output at different frequency settings

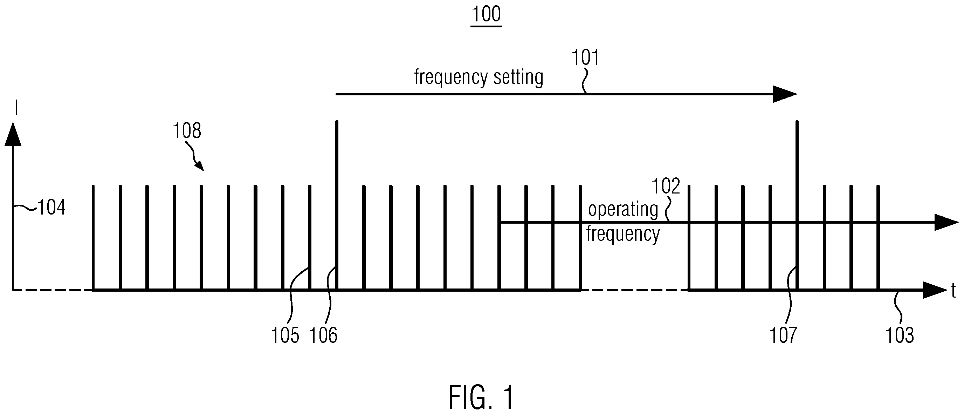

**Fig. 1** illustrates an exemplary modulation 100 of a series 108 of light emission pulses 105, 106, 107 of an exemplary laser diode unit / laser diode(s) (not shown), with the ordinate axis 104 exemplary representing the amplitude or energy of the light pulses 105, 106, 107 or exemplary representing the electrical current(s) provided to the laser diode unit / laser diode that triggers / trigger said light pulses 105, 106, 107, and with the abscissa axis 103 exemplary representing time.

The exemplary pulses denoted with reference numerals 106 and 107 exemplary represent pulses emitted by the laser diode unit / laser diode(s) with an amplitude / energy that are required or sufficient to trigger light emission of a/the solid-state laser (not shown) that is receiving said pulses from the laser diode unit / laser diode(s), i.e. said pulses 106, 107 are operated at or above the lasing threshold of the solid-state laser and with a frequency 101 corresponding to a/the requested frequency setting of the pulsed solid-state laser.

Furthermore, the reference numeral 102 exemplary denotes an/the operating frequency of the exemplary laser diode unit / laser diode(s) with which the laser diode unit / laser diode(s) emits light pulses 105, 106, 107 to pump the solid-state laser and wherein the light pulses 105 with a frequency different from a/the requested frequency setting 101 are operated to not trigger light emission of the solid-state laser, i.e. are operated with an amplitude / energy below the lasing threshold of the solid-state laser.

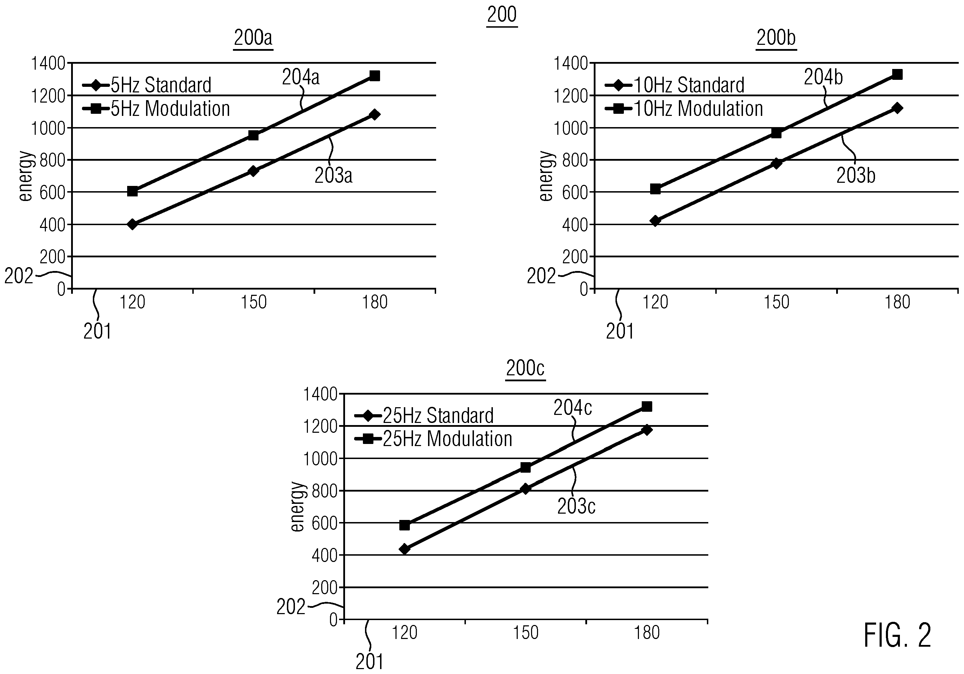

**Fig. 2** exemplary illustrates test results 200 comparing the solid-state laser power/energy output at different requested frequency settings 200a, 200b, 200c comprising the requested frequency settings of 5 Hz, 10 Hz and 25 Hz when using standard pumping methods 203a, 203b, 203c as compared to pumping methods of a solid-state laser with a laser diode unit / laser diode(s) according to the herein described means and methods.

For example, the panel 200a shows the solid-state laser power/energy output 202 at a requested solid-state laser frequency setting of 5 Hz in dependence of the electrical current 201 supplied to a/the laser diode unit / laser diode(s) for a standard pumping 203a (line connecting the diamond-shaped measurement points) and for a modulated pumping 204a according to the method exemplary described herein (line connecting the square-shaped measurement points).

The panel 200b exemplary shows the solid-state laser power/energy output 202 at a requested solid-state laser frequency setting of 10 Hz in dependence of the electrical current 201 supplied to a/the laser diode unit / laser diode(s) for a standard pumping 203b (line connecting the diamond-shaped measurement points) and for a modulated pumping 204b according to the method exemplary described herein (line connecting the square-shaped measurement points).

The panel 200c exemplary shows the solid-state laser power/energy output 202 at a requested solid-state laser frequency setting of 25 Hz in dependence of the electrical current 201 supplied to a/the laser diode unit / laser diode(s) for a standard pumping 203c (line connecting the diamond-shaped measurement points) and for a modulated pumping 204c according to the method exemplary described herein (line connecting the square-shaped measurement points).

As can be seen when comparing the three different panels 200a, 200b, 200c which have the same scales, the gains/improvements in solid-state laser power/energy output efficiencies are significant for all requested frequency settings but highest for lower requested frequency settings.

For completeness, it is noted that for the shown results the laser diode unit / laser diode(s) for exemplary pumping the solid-state laser according to the method exemplary described herein, the exemplary operating frequency of the laser diode unit / laser diode(s) was 100 Hz.

However, the significant gains in laser power/energy output of a/the solid-state laser can be achieved also with other operating frequencies of the laser diode unit / laser diode(s).

Followed by two sheets comprising Fig.1 and Fig. 2, wherein the reference numerals identify the following components.
**100** exemplary modulation of light pulses for pumping a solid-state laser
**101** exemplary (requested) frequency / frequency setting of a/the solid-state laser
**102** exemplary operating frequency of exemplary laser diode unit / exemplary laser diode(s)
**103** exemplary abscissa axis, exemplary time axis, t
**104** exemplary ordinate axis, exemplary representing amplitude / energy of light pulses emitted by exemplary laser diode unit / exemplary laser diode(s) or exemplary electrical current, l, supplied to the laser diode unit / laser diode(s)
**105** exemplary light pulse(s) emitted by exemplary laser diode unit / exemplary laser diode(s) that are operated with amplitudes/energies that do not trigger light emission by the solid-state laser, i.e. exemplary laser diode unit / exemplary laser diode pulses for pumping the solid-state laser that are operated below the lasing threshold of the solid-state laser
**106, 107** exemplary light pulse(s) emitted by exemplary laser diode unit / exemplary laser diode(s) that are operated with sufficient high amplitudes/energies that trigger light emission by the solid-state laser, i.e. exemplary laser diode unit / exemplary laser diode pulses for pumping the solid-state laser that are operated at or above the lasing threshold of the solid-state laser
**108** exemplary series of light pulses emitted by exemplary laser diode unit / laser diode(s) for pumping a/the solid-state laser
**200** exemplary test results for achieved gains in laser power output of a/the solid-state laser in pulsed mode when pumped according to the herein described method as compared to standard pumping methods
**200a, 200b, 200c** exemplary test results for requested frequency settings 5 Hz, 10 Hz and 25 Hz of solid-state laser
**201** exemplary abscissa axis, exemplary electrical current supplied to exemplary laser diode unit / exemplary laser diode(s), for example in units of Ampere
**202** exemplary ordinate axis, exemplary laser power/energy output of exemplary solid-state laser in pulsed mode, for example specified in millijoule (mJ)
**203a** exemplary laser power/energy output of solid-state laser for standard pumping and a requested frequency setting of 5 Hz for the solid-state laser
**204a** exemplary laser power/energy output of solid-state laser for pumping according to the herein described method and a requested frequency setting of 5 Hz for the solid-state laser
**203b** exemplary laser power/energy output of solid-state laser for standard pumping and a requested frequency setting of 10 Hz for the solid-state laser
**204b** exemplary laser power/energy output of solid-state laser for pumping according to the herein described method and a requested frequency setting of 10 Hz for the solid-state laser
**203c** exemplary laser power/energy output of solid-state laser for standard pumping and a requested frequency setting of 25 Hz for the solid-state laser
**204c** exemplary laser power/energy output of solid-state laser for pumping according to the herein described method and a requested frequency setting of 25 Hz for the solid-state laser

## Claims

1. Method for operating a pulsed diode-pumped solid-state Tm:YAG laser comprising:
providing a pump light source for pumping a Tm:YAG laser, said pump light source comprising at least one laser diode unit configured for emitting a series of light pulses (105, 106, 107) for pumping the Tm:YAG laser, wherein the at least one laser diode unit is emitting light pulses with a wavelength between 778 to 782 nm and with pulse durations of up to 500 µs and with amplitudes of up to a maximum current of 250 A, and wherein said at least one laser diode unit is emitting light emission pulses (106,107) with a pulse repetition frequency (101) close to or equal to a requested frequency setting of the Tm:YAG laser and is emitting other light emission pulses (105) with frequencies not equal to the requested frequency setting of the Tm:YAG laser, i.e. is emitting other light emission pulses (105) with frequencies outside a predetermined interval around the requested frequency setting of the Tm:YAG laser,
modulating the series of light emission pulses (105, 106, 107) of the at least one laser diode unit such that only the light pulses (106, 107) with a frequency (101) close to or equal to the requested frequency setting of the Tm:YAG laser are operated with a required pulse amplitude and/or a required pulse duration sufficient to trigger light emission of the Tm:YAG laser, wherein the requested diode-pumped Tm:YAG laser frequency setting lies at a frequency below 300 Hz,
and such that said other light pulses (105) emitted by the at least one laser diode unit are operated with a pulse amplitude and/or pulse duration not sufficient to trigger light emission of the Tm:YAG laser.

2. Method according to claim 1, wherein the requested diode-pumped Tm:YAG laser frequency setting lies at a frequency at or below 100 Hz.

3. Method according to one of the preceding claims, wherein the requested laser frequency setting for the diode-pumped Tm:YAG laser is one of the following frequency settings: 5, 10, 20, or 25 Hz,
and wherein the at least one laser diode unit is operated to emit light pulses (105, 106, 107) with a frequency (102) of 100 Hz, and wherein for a requested Tm:YAG laser frequency setting of 5 Hz, only every 20^{th} light pulse of the laser diode unit is operated with the required pulse amplitude and/or the required pulse duration to trigger light emission of the Tm:YAG laser,
and wherein for a requested Tm:YAG laser frequency setting of 10 Hz only every 10^{th} light pulse of the laser diode unit is operated with the required pulse amplitude and/or the required pulse duration to trigger light emission of the Tm:YAG laser,
and wherein for a requested Tm:YAG laser frequency setting of 20 Hz only every 5^{th} light pulse of the laser diode unit is operated with the required pulse amplitude and/or the required pulse duration to trigger light emission of the Tm:YAG laser,
and wherein for a requested Tm:YAG laser frequency setting of 25 Hz only every 4^{th} light pulse of the laser diode unit is operated with the required pulse amplitude and/or the required pulse duration to trigger light emission of the Tm:YAG.

4. Method according to one of the preceding claims, wherein during operation of the pulsed diode-pumped Tm:YAG laser, the electrical current provided to drive the at least one laser diode unit is non-zero at all times.

5. Diode-pumped Tm:YAG laser system comprising:
a Tm:YAG laser,
at least one laser diode unit configured to operate according to one of the preceding claims.

6. Diode-pumped Tm:YAG laser according to the preceding laser system claim, wherein the at least one laser diode unit comprises a single laser diode or a one-dimensional array of laser diodes, e.g. a diode-laser bar, or a two-dimensional array of laser diodes, e.g. a stack of diode-laser bars, or a three-dimensional array of laser diodes, e.g. comprising multiple stacks of diode-laser bars.

## Patentansprüche

1. Verfahren zum Betreiben eines gepulsten diodengepumpten Tm:YAG-Festkörperlasers, das umfasst:
Bereitstellen einer Pumplichtquelle zum Pumpen eines Tm:YAG-Lasers, wobei die Pumplichtquelle wenigstens eine Laserdioden-Einheit umfasst, die zum Emittieren einer Reihe von Lichtimpulsen (105, 106, 107) zum Pumpen des Tm:YAG-Lasers ausgeführt ist, die wenigstens eine Laserdioden-Einheit Lichtimpulse mit einer Wellenlänge zwischen 778 und 782 nm und mit Impulsdauern von bis zu 500 µs sowie mit Amplituden bis zu einem maximalen Strom von 250 A emittiert, und die wenigstens eine Laserdioden-Einheit Lichtemissionsimpulse (106, 107) mit einer Impulswiederholfrequenz (101) emittiert, die nahe oder auf einer erforderlichen Frequenzeinstellung des Tm:YAG-Lasers liegt, und andere Lichtemissionsimpulse (105) mit Frequenzen emittiert, die nicht auf der erforderlichen Frequenzeinstellung des Tm:YAG-Lasers liegen, d.h. andere Lichtemissionsimpulse (105) mit Frequenzen emittiert, die außerhalb eines vorgegebenen Intervalls um die gewünschte Frequenzeinstellung des Tm:YAG-Lasers herum liegen,
Modulieren der Reihe von Lichtemissionsimpulsen (105, 106, 107) der wenigstens einen Laserdioden-Einheit so, dass nur die Lichtimpulse (106, 107) mit einer Frequenz (101), die nahe oder auf der gewünschten Frequenzeinstellung des Tm:YAG-Lasers liegen, mit einer erforderlichen Impulsamplitude und/oder einer erforderlichen Impulsdauer betrieben werden, die ausreichen/ausreicht, um Lichtemission des Tm:YAG-Lasers auszulösen, wobei die erforderliche Frequenzeinstellung des diodengepumpten Tm:YAG-Lasers bei einer Frequenz unter 300 Hz liegt, und so, dass die anderen Lichtimpulse (105), die von der wenigstens einen Laserdioden-Einheit emittiert werden, mit einer Impulsamplitude und/oder Impulsdauer betrieben werden, die nicht ausreichen/ausreicht, um Lichtemission des Tm:YAG-Lasers auszulösen.

2. Verfahren nach Anspruch 1, wobei die erforderliche Frequenzeinstellung des diodengepumpten Tm:YAG-Lasers bei einer Frequenz von 100 Hz oder darunter liegt.

3. Verfahren nach einem der vorangehenden Ansprüche, wobei die erforderliche Laserfrequenzeinstellung für den diodengepumpten Tm:YAG-Laser eine der folgenden Frequenzeinstellungen ist:
5, 10, 20, oder 634, 734 Hz,
und wobei die wenigstens eine Laserdioden-Einheit so betrieben wird, dass sie Lichtimpulse (105, 106, 107) mit einer Frequenz (102) von 100 Hz emittiert, für eine erforderliche Tm:YAG-Laserfrequenzeinstellung von 5 Hz nur jeder zwanzigste Lichtimpuls der Laserdioden-Einheit mit der zum Auslösen von Lichtemission des Tm:YAG-Lasers erforderlichen Impulsamplitude und/oder erforderlichen Impulsdauer betrieben wird, für eine erforderliche Tm:YAG-Laserfrequenzeinstellung von 10 Hz nur jeder zehnte Lichtimpuls der Laserdioden-Einheit mit der zum Auslösen von Lichtemission des Tm:YAG-Lasers erforderlichen Impulsamplitude und/oder erforderlichen Impulsdauer betrieben wird, für eine erforderliche Tm:YAG-Laserfrequenzeinstellung von 20 Hz nur jeder fünfte Lichtimpuls der Laserdioden-Einheit mit der zum Auslösen von Lichtemission des Tm:YAG-Lasers erforderlichen Impulsamplitude und/oder erforderlichen Impulsdauer betrieben wird, und für eine erforderliche Tm:YAG-Laserfrequenzeinstellung von 25 Hz nur jeder vierte Lichtimpuls der Laserdioden-Einheit mit der zum Auslösen von Lichtemission des Tm:YAG-Lasers erforderlichen Impulsamplitude und/oder erforderlichen Impulsdauer betrieben wird.

4. Verfahren nach einem der vorangehenden Ansprüche, wobei bei Betrieb des gepulsten diodengepumpten Tm:YAG-Lasers der zum Ansteuern der wenigstens einen Laserdioden-Einheit bereitgestellte elektrische Strom jederzeit ungleich Null ist.

5. Diodengepumptes Tm:YAG-Lasersystem, das umfasst:
einen Tm:YAG-Laser, wenigstens eine Laserdioden-Einheit, die so ausgeführt ist, dass sie nach einem der vorangehenden Ansprüche arbeitet.

6. Diodengepumpter Tm:YAG-Laser nach dem vorangehenden Lasersystem-Anspruch, wobei die wenigstens eine Laserdioden-Einheit eine einzelne Laserdiode oder eine eindimensionale Anordnung von Laserdioden, beispielsweise einen Dioden-Laserstab, oder eine zweidimensionale Anordnung von Laserdioden, beispielsweise einen Stapel von Dioden-Laserstäben, oder eine dreidimensionale Anordnung von Laserdioden umfasst, die beispielsweise mehrere Stapel von Dioden-Laserstäben umfasst.

## Revendications

1. Procédé de fonctionnement d'un laser Tm:YAG à état solide pulsé pompé par diode, comprenant :
la fourniture d'une source de lumière de pompage pour pomper un laser Tm:YAG, ladite source de lumière de pompage comprenant au moins une unité de diode laser configurée pour émettre une série d'impulsions de lumière (105, 106, 107) pour pomper le laser Tm:YAG, dans lequel ladite au moins une unité de diode laser émet des impulsions de lumière avec une longueur d'onde comprise entre 778 et 782 nm et avec des durées d'impulsion allant jusqu'à 500 µs et des amplitudes allant jusqu'à un courant maximal de 250 A, et dans lequel ladite au moins une unité de diode laser émet des impulsions d'émission de lumière (106, 107) avec une fréquence de répétition d'impulsion (101) proche ou égale à un réglage de fréquence requis du laser Tm:YAG et émet d'autres impulsions d'émission de lumière (105) à des fréquences non égales au réglage de fréquence requis du laser Tm:YAG, c'est-à-dire qu'elle émet d'autres impulsions d'émission de lumière (105) à des fréquences qui sont hors d'un intervalle prédéterminé autour du réglage de fréquence requis du laser Tm:YAG,
la modulation de la série d'impulsions d'émission de lumière (105, 106, 107) de ladite au moins une unité de diode laser de telle sorte que seules les impulsions de lumière (106, 107) à une fréquence (101) proche ou égale au réglage de fréquence requis du laser Tm:YAG sont utilisées avec une amplitude d'impulsion requise et/ou une durée d'impulsion requise suffisante pour déclencher une émission de lumière du laser Tm:YAG, dans lequel le réglage de fréquence requis du laser Tm:YAG pompé par diode est une fréquence inférieure à 300 Hz, et de telle sorte que lesdites autres impulsions de lumière (105) émises par ladite au moins une unité de diode laser sont utilisées avec une amplitude d'impulsion et/ou une durée d'impulsion qui n'est pas suffisante pour déclencher une émission de lumière du laser Tm:YAG.

2. Procédé selon la revendication 1, dans lequel le réglage de fréquence requis du laser Tm:YAG pompé par diode est une fréquence inférieure ou égale à 100 Hz.

3. Procédé selon l'une des revendications précédentes, dans lequel le réglage de fréquence requis pour le laser Tm:YAG pompé par diode est un réglage parmi les réglages de fréquence suivants : 5, 10, 20 et 25 Hz,
et dans lequel ladite au moins une unité de diode laser est utilisée pour émettre des impulsions de lumière (105, 106, 107) à une fréquence (102) de 100 Hz, et dans lequel, pour un réglage de fréquence requis du laser Tm:YAG de 5 Hz, seule chaque 20^{ième} impulsion de lumière de l'unité de diode laser est utilisée avec l'amplitude d'impulsion requise et/ou la durée d'impulsion requise pour déclencher une émission de lumière du laser Tm:YAG,
et dans lequel, pour un réglage de fréquence requis du laser Tm:YAG de 10 Hz, seule chaque 10^{ième} impulsion de lumière de l'unité de diode laser est utilisée avec l'amplitude d'impulsion requise et/ou la durée d'impulsion requise pour déclencher une émission de lumière du laser Tm:YAG,
et dans lequel, pour un réglage de fréquence requis du laser Tm:YAG de 20 Hz, seule chaque 5^{ième} impulsion de lumière de l'unité de diode laser est utilisée avec l'amplitude d'impulsion requise et/ou la durée d'impulsion requise pour déclencher une émission de lumière du laser Tm:YAG,
et dans lequel, pour un réglage de fréquence requis du laser Tm:YAG de 25 Hz, seule chaque 4^{ième} impulsion de lumière de l'unité de diode laser est utilisée avec l'amplitude d'impulsion requise et/ou la durée d'impulsion requise pour déclencher une émission de lumière du laser Tm:YAG.

4. Procédé selon l'une des revendications précédentes, dans lequel, durant l'utilisation du laser Tm:YAG pulsé pompé par diode, le courant électrique fourni pour alimenter ladite au moins une unité de diode laser est non nul à tout moment.

5. Système de laser Tm:YAG pompé par diode, comprenant :
un laser Tm:YAG,
au moins une unité de diode laser configurée pour fonctionner selon l'une des revendications précédentes.

6. Laser Tm:YAG pompé par diode selon la revendication de système de laser qui précède, dans lequel ladite au moins une unité de diode laser comprend une diode laser unique ou un réseau unidimensionnel de diodes laser, par exemple une barre laser à diodes, ou un réseau bidimensionnel de diodes laser, par exemple une pile de barres laser à diodes, ou un réseau tridimensionnel de diodes laser, par exemple de multiples piles de barres laser à diodes.
